# EUROPEAN PATENT APPLICATION

(11) **EP 4 623 954 A1**
(43) Date of publication of application: **01.10.2025**
(21) Application number: 23897788.8
(22) Date of filing: 28.11.2023
(51) Int. Cl.: A61M 5/152

(54) **BALLOON FOR BALLOON INFUSER**

(30) Priority: 29.11.2022 JP 2022190708
(71) Applicant: Nipro Corporation, Osaka 566-8510 (JP)
(72) Inventor: UENISHI, Masayuki, Settsu-shi, Osaka 566-8510 (JP); ISHIKURA, Kohzo, Settsu-shi, Osaka 566-8510 (JP)
(74) Representative: Germain Maureau
(86) International application number: PCT/JP2023/042608
(87) International publication number: WO 2024/117140

(57) **Abstract**

A balloon 100 for a balloon infuser includes: a balloon body 101 that is insertable into a shell 201 and reserves and pumps out a drug solution; a drug solution passage 120 fixed to one end of the balloon body 101 and provided with a flow path through which the drug solution is introduced into and discharged from the balloon body 101; and a connector 123 detachably connecting the ballon to the shell 201 while the ballon is located in the shell 201. The balloon body 101 is removable from the shell 201 by disconnecting the connector 123 from the shell 201.

## Description

### TECHNICAL FIELD

The present disclosure relates to a balloon for a balloon infuser and a balloon infuser using the balloon.

### BACKGROUND ART

Small amounts of drug, such as an anesthetic, an analgesic, an anticancer drug, or an antibiotic, may be continuously injected into a body over time. For continuous injection of the drug into the body, there is a method using a balloon infuser. The balloon infuser includes a balloon made of an elastic material and can continuously pump out a drug, which has been injected into the balloon, by means of the contraction of the balloon (see, e.g., Patent Document 1). The balloon infuser has a simple structure as compared with a mechanical pump or other means, requiring no power source, for example, and can be handled easily.

### CITATION LIST

### PATENT DOCUMENT

PATENT DOCUMENT 1: Japanese Unexamined Patent Publication No. H7-116250

### SUMMARY OF THE DISCLOSURE

### TECHNICAL PROBLEMS

The balloon infuser employs a structure in which a shell covers a balloon in case where the balloon is damaged. There are two types of shells, namely, a hard shell with shape retention, and a soft shell. A hard shell has a high strength which ensures a high safety, but has the problem of a large volume of medical waste due to its bulkiness. A soft shell has no shape retention and is thus not bulky, but has low strength and is thus damaged easily. If damaged, a soft shell needs to be discarded including the balloon that is not damaged.

It is an object of the present disclosure to solve the problem of increased medical waste out of balloon infusers.

### SOLUTIONS TO THE PROBLEMS

A balloon for a balloon infuser according to a first aspect of the present disclosure includes: a balloon body that is insertable into a shell and reserves and pumps out a drug solution; a drug solution passage fixed to one end of the balloon body and provided with a flow path through which the drug solution is introduced into and discharged from the balloon body; and a connector detachably connecting the ballon body to the shell while the ballon body is located in the shell. The balloon body is removable from the shell by disconnecting the connector from the shell.

In the balloon for a balloon infuser according to the first aspect, the balloon body is holdable in the shell by connecting the connector to the shell, and removable from the shell by disconnecting the connector. Accordingly, the balloon body can be filled with a drug solution and used, while being inserted into the shell and protected. After use, the balloon body can be pulled out of the shell and discarded. As a result, only the balloon becomes the medical waste, which can reduce an increase in the medical waste. If damaged, only a soft shell may be replaced, which can reduce the amount of waste. If the connector of a universal cap employed in a PET bottle for beverages or other items made of polyethylene terephthalate (PET) is used as the connector of the balloon, the shell can be replaced with another container, such as the PET bottle, in case of emergency lacking the shell.

In the balloon for a balloon infuser according to the first aspect, the connector may have a guide structure that guides movement relative to the shell in an axial direction. With this configuration, the connector can move smoothly relative to the shell, when the connector is connected to the shell and when the connector is disconnected and the balloon body is pulled out, which can improve the handleability.

In the balloon according to the first aspect, the guide structure may include a connector-side screw to be threadedly engaged with an opening-side screw at the opening of the shell. With this configuration, firm connection and easier disconnection can be provided. The guide structure does not necessarily include the screws but may be a combination of a projection and a groove.

In the balloon for a balloon infuser according to the first aspect, the connector may have a lock mechanism that restricts disconnection from the opening of the shell. With this configuration, unintentional disconnection of the connector can be reduced. The lock mechanism may be disconnected from the opening of the shell, for example, by deforming or riding over a locking projection under application of a force. With this configuration, careless disconnection can be reduced. Other specific examples of this lock mechanism include a projection that can go beyond a screw thread, an L-shaped or crank-shaped groove, a groove tapered to a distal end with an increasing friction, a structure in which a projection of a tongue piece goes beyond a projection of the counterpart. The lock mechanism may notify the user of completion of locking through a click feeling.

In the balloon for a balloon infuser according to the first aspect, the connector may include a flexible piece and a projection at a free end of the flexible piece. The connector may be connected to the shell by bringing the connector close to the shell so that the projection rides over a shell-side projection of the shell. With this configuration, the balloon can be connected to the shell in a one-touch operation, which improves the handleability largely. The flexible piece may be a tongue-shaped portion extending downward, or may be a cut-out of the connector to make the connector flexible. Depending on the material and shape of the connector, the flexible piece may be excluded. Alternatively, not the projection of the connector but the shell-side projection may have the flexible piece. In this case, the connector is mountable inside the shell. In addition, the connector-side projection and/or the shell-side projection may have an inclined surface so that the projections can ride over each other. Alternatively, the flexible piece may have a lever, for example, to be able to displace the projection at the time of attachment and detachment. In this case, the flexible piece may have no projection but a recess to be engaged with the projection of the shell.

In the balloon for a balloon infuser according to the first aspect, the connector may be integrally molded with the drug solution passage. With this configuration, the balloon can be easily connected to the shell.

**In** the balloon for a balloon infuser according to the first aspect, the connector may be separable from the drug solution passage. With this configuration, the connector is made reusable, which can further reduce the waste.

A second aspect of the present disclosure is directed to a balloon for a balloon infuser used in combination with a shell and a connector. The balloon includes: a balloon body that is insertable into a shell and reserves and pumps out a drug solution; and a drug solution passage fixed to one end of the balloon body and provided with a flow path through which the drug solution is introduced into and discharged from the balloon body. The drug solution passage includes a portion sandwiched between the shell and the connector. The balloon body is held inside the shell by connecting the connector to the shell. The balloon body is removable from the shell by disconnecting the connector from the shell.

**In** the balloon for a balloon infuser according to the second aspect, the shell and the connector are used in combination and become reusable, which can further reduce the waste.

The sandwiched portion may be a flange, for example, extending in the radial direction and sandwiched between the connector and the shell in the vertical direction. The connector may include, for example, a guide structure that guides movement relative to the shell in an axial direction, a connector-side screw to be threadedly engaged with an opening-side screw at the opening of the shell, a lock mechanism that restricts disconnection from the opening of the shell, and a flexible piece connectable to the shell.

The balloon for a balloon infuser according to the first and second aspects may further include: an indicator that indicates characters indicating at least one of a lot number, an item number, a capacity of the balloon body, or a flow rate. The characters may be a numerical value representing a total number, for example. This configuration allows the balloon to be easily identified.

The balloon for a balloon infuser according to the first and second aspects may further include: an identification correspondence portion corresponding to an identifier in the shell. The identifier and the identification correspondence portion indicate that at least a capacity of the balloon body matches a volume of the shell. The identification correspondence portion may be an identification indicator corresponding to the identifier, or may indicate whether the size of the connection partner matches. The identifier and the identification indicator may be of any of various types that are visually checkable and may be in the same color, the same characters, and/or the same symbol. The characters may be a numerical value representing a total number, for example. The symbol may be a circle, a triangle, or a cross, for example. This configuration enables the assembly to the shell having a capacity corresponding to the capacity of the balloon body, and can encourage the user not to erroneously use the balloon in combination with a shell that is too small to inflate sufficiently. This configuration can also reduce the possible burst of the balloon due to the interference between the shell and the balloon.

The identifier and the identification correspondence portion may have the following configurations. For example, the lines are aligned or the items in the same shape and/or color are aligned when the balloon infuser is attached to the proper shell. The indicators by means of the labels, for example, are not necessarily the same. For example, one indicator may be a line, with which the other indicator may be aligned. The indications may be each made by attaching a label, printing on the surface of a member, or coloring the member. The balloon body may include a movable portion that moves in accordance with the amount of the injected drug solution. The approximate amount of the drug solution may be indicated by the point where a scale on the shell and a gridline on the movable portion overlap each other. In this case, whether the combination is proper can be shown by matching the colors of the scale and the gridline.

The balloon according to the first and second aspects may further include: a used-state indicator that appears when the connector is connected to or disconnected from the shell, and indicates that the balloon has been used. With this configuration, the user can be encouraged not to reuse the balloon. Specific examples of the used-state indicator may include the following configurations. A member, such as a pin, is pushed in and appears on the surface of the connector. Pressure-sensitive paper is attached on which another color appears upon application of pressure. A part is broken, for example, by connection or disconnection. A coating is removed by connection or disconnection so that the indicator appears.

In this case, the used-state indicator may have a detent mechanism that restricts return of the usage indicator. This can further reduce possible reuse.

A balloon infuser according to an aspect of the present disclosure includes: a shell; a balloon for the balloon infuser, the balloon including a balloon body that reserves and pumps out a drug solution, a drug solution passage fixed to one end of the balloon body and provided with a flow path through which the drug solution is introduced into and discharged from the balloon body, and. a connector detachably connecting the ballon body to the shell while the ballon body is located in the shell. The balloon body is removable from the shell by disconnecting the connector from the shell.

### ADVANTAGES OF THE INVENTION

The balloon for a balloon infuser according to the present disclosure provides a balloon infuser causing less increase in the medical waste.

### BRIEF DESCRIPTION OF THE DRAWINGS

[FIG. 1] FIG. 1 is a side view of a balloon infuser according to an embodiment.
[FIG. 2] FIG. 2 is a cross-sectional view of an enlarged part of the balloon infuser according to the embodiment.
[FIG. 3] FIG. 3 is a side view of a variation of a balloon.
[FIG. 4] FIG. 4 is a cross-sectional view of a shell combinable with the balloon of FIG. 3.
[FIG. 5] FIG. 5 is a partial cross-sectional view of a process of connecting the balloon to the shell according to the variation.
[FIG. 6] FIG. 6 is a side view of a balloon infuser including the shell according to the variation.
[FIG. 7] FIG. 7 is a cross-sectional view of a balloon infuser including a cap according to the variation.
[FIG. 8] FIG. 8 is a cross-sectional view of a balloon infuser including a used-state indicator.

### DESCRIPTION OF EMBODIMENTS

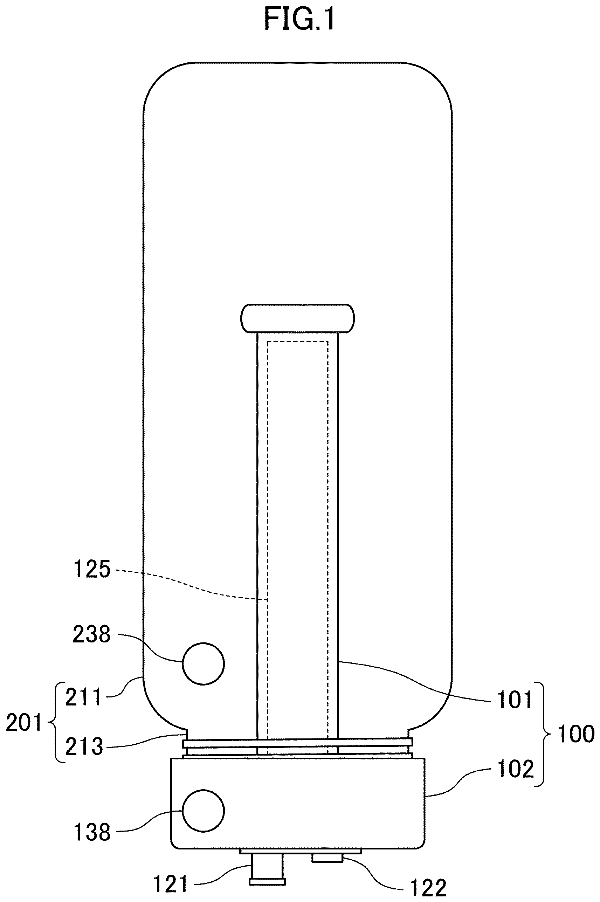

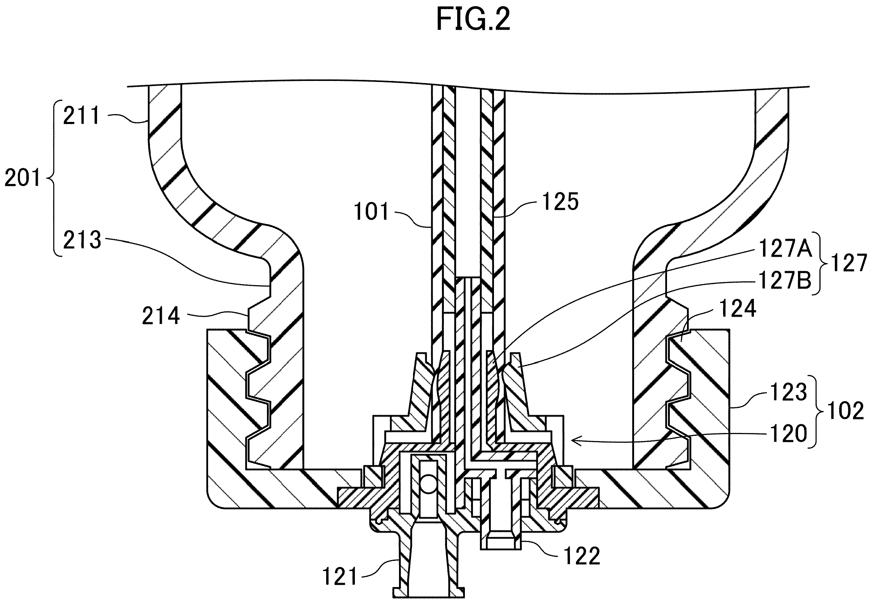

As shown in FIGS. 1 and 2, a balloon 100 for a balloon infuser according to an embodiment includes a balloon body 101 and a cap 102 to which a proximal end of the balloon body 101 is fixed. The balloon body 101 is a tubular body made of an elastic material with the distal end away from the cap 102 closed.

The cap 102 is provided with a drug solution passage 120 with an inlet port 121 and an outlet port 122. A drug solution can be introduced through the inlet port 121 into the balloon body 101. The inlet port 121 is provided with a check valve. When the outlet port 122 is closed, the drug solution introduced through the inlet port 121 is reserved in the balloon body 101. Being made of an elastic material, the balloon body 101 is inflated by the drug solution introduced through the inlet port 121, and a pressure is applied to the drug solution in the balloon body 101. Thus, once the outlet port 122 is open, the drug solution in the balloon body 101 is led out through the outlet port 122 by the internal pressure. By connecting an orifice or the like for controlling the flow path to the outlet port 122, the drug solution in the balloon body 101 can be continuously led out at a predetermined flow rate.

The cap 102 has a connector 123 that is detachably connected to the opening 213 of a shell 201. Once the balloon body 101 is inserted into the shell 201 through the opening 213 and the connector 123 is connected to the opening 213, the balloon infuser becomes usable as a balloon infuser including a shell. After use, the connector 123 is detached from the opening 213, and the balloon body 101 can be pulled out of the shell 201.

The balloon 100 for a balloon infuser according to this embodiment can be used as a balloon infuser with the balloon body 101 filled with a drug solution with the balloon body 101 inserted into the shell 201. On the other hand, after use, the balloon body can be pulled out of the shell 201, and only the balloon 100 can be discarded, which can significantly reduce the amount of waste. While the reuse of the shell 201 can further reduce the waste, even the shell 201 for one-way use separated from the balloon 100 can be handled as a general waste, which can reduce the medical waste.

FIG. 1 shows an example where the shell 201 is a hard shell with shape retention. The hard shell has a high strength which ensures a high safety, and is also advantageous in reducing erroneous use, such as pressing the balloon body 101 with a hand and increasing the amount of the drug solution to be led out. The shell 201 may be however a soft shell. If damaged before use, only a soft shell may be replaced and there is no need to discard the balloon 100, which can reduce the amount of waste. The soft shell is reusable and a soft shell separated from the balloon 100 can be handled as a general waste, which can reduce the medical waste.

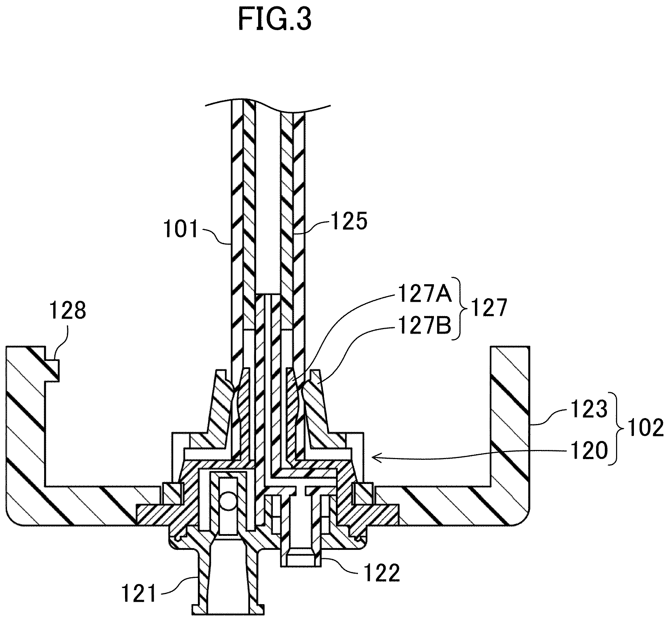

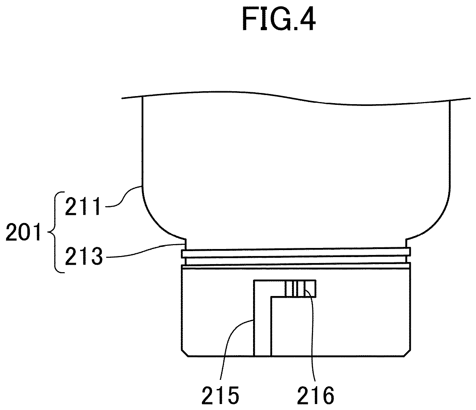

The connector 123 and the opening 213 may have various configurations that can be detachably engaged with each other. For example, the connector 123 may have a guide structure that guides the movement relative to the shell 201 in the axial direction and stop at a target point. An example of such a guide structure is described in this embodiment where the connector 123 has a connector-side screw 124 and the opening 213 has an opening-side screw 214. Being rotated and threadedly engaged with or disengaged from the opening 213, the connector 123 moves relative to the shell 201 in the axial direction and is held temporarily. Not limited to the screw, the guide structure may include a groove and a projection to be engaged with the groove. For example, as shown in FIGS. 3 and 4, the opening 213 may have a groove 215, and the connector 123 may have a projection 128. The connector 123 may have a groove, and the opening 213 may have a projection.

Alock mechanism may be provided to reduce unintentional disconnection between the connector 123 and the opening. In the case of threadedly engaging the connector 123 and the opening 213 using screws, for example, a projection that can ride over in two directions is provided near the distal end of the screw thread. This can be used as a lock mechanism. This configuration can avoid unintentional disconnection, while provide a click feeling when the screw is tightened to a predetermined angle. This helps reduce the risk of damage caused by over-tightening of the screw, as well as the occurrence of detachment of the screw due to forgetting to tighten the screw, or other problems.

**In** the case of combining the groove 215 and the projection 128, the vicinity of the **L-**shaped distal end of the groove 215 shown in FIG. 3 functions as a lock mechanism that can lock the connector 123 and the opening 213 at locations relatively close to each other. The groove 215 may have a crank to serve as a lock mechanism. The groove 215 may have a protrusion 216 over which the projection 128 can ride in two directions. **In** this case, the user can be notified of the locking through a click feeling. The lock mechanism may be obtained by reducing the width of the groove near the distal end of the groove 215 to increase friction. The configuration in which the protrusion 216 is provided or the width of the groove is reduced is also effective when the groove is linear. The same or similar lock can be achieved even if the connector 123 has a groove.

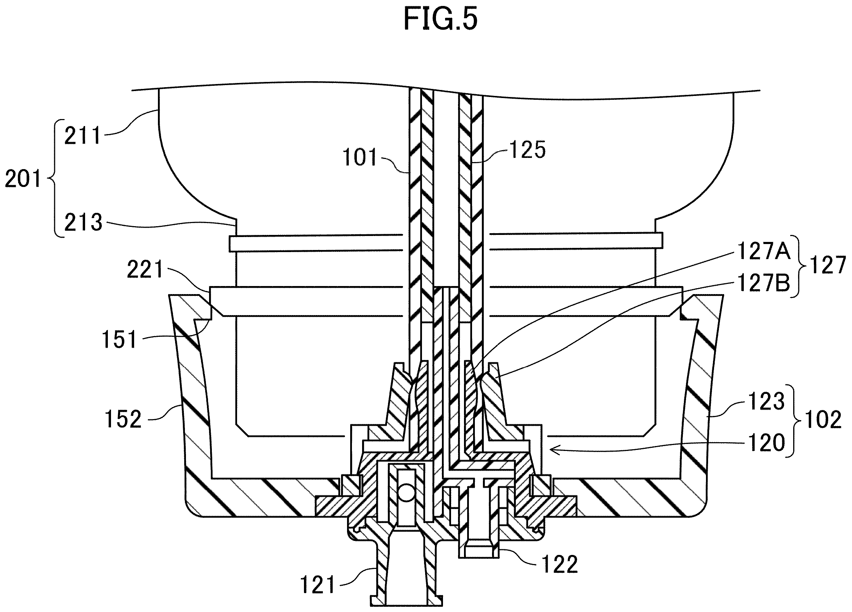

As a configuration capable of detachably engaging the connector 123 and the opening 213, a configuration can be employed in which one of the two has a projection and the other has a projection that can ride over the projection in two directions. For example, as shown in FIG. 5, the connector 123 may have a projection 151, and the opening 213 may have a projection 221. By bringing the connector 123 close to the opening 213, the projection 151 rides over the projection 221 and the connector 123 is attached to and locked in the opening 213.

In FIG. 5, the projection 151 is located at the free end of a flexible piece 152 formed by cutting out a part of the side surface of the connector 123. Accordingly, when the projection 151 rides over the projection 221, the flexible piece 152 warps to allow the projection 151 easily ride over the projection 221. A configuration without the flexible piece 152 may also be adopted depending on the material or shape of the connector 123.

In FIG. 5, the upper end surface of the projection 151 and the lower end surface of the projection 221 are inclined. Accordingly, when the connector 123 is attached to the opening 213, the projection 151 can easily ride over the projection 221. Note that only one of the projection 151 or the projection 221 may have an inclined surface, or neither of the protrusions may have an inclined surface.

In FIG. 5, the lower end surface of the projection 151 and the upper end surface of the projection 221 are flat. Accordingly, the projection 151 has difficulty in riding over the projection 221 in a direction of disconnecting the connector 123 from the opening 213, which causes less unintentional disconnection of the connector 123. In order to disconnect the connector 123, the projection 151 can ride over the projection 221 with the flexible piece 152 slightly pressed and warped outward with a finger. Depending on the required engaging force, abutting surfaces at the time of disconnection may be inclined.

The flexible piece may be a tongue-shaped portion extending downward, or may be a cut-out of the connector to make the connector flexible. Alternatively, the flexible piece may have a lever. When pressed, the lever causes displacement, such as a rise, of the projection, which leads to the disconnection. This facilitates the attachment and detachment and can reduce unintentional fall-off.

Not only the combination of the flexible piece and the lever, but also various configurations which cause some deformation when a force is applied may be employed. For example, a protrusion which is deformed when a force is applied may be provided. This protrusion may be engaged with another member so as to cause locking, and deformed when a force is applied so as to cause unlocking.

While the flexible piece 152 has the projection 151 of the connector 123 and the projection 221 of the opening 213 is in the shape of a fixed flange in FIG. 5, the opening 213 may have a flexible piece and the connector 123 may have a fixed projection. Alternatively, one of the projections may be a recess, and the other projection and this recess may be engaged with each other.

As a mechanism for detachably engaging the connector 123 and the opening 213, engagement by means of friction between tapered surfaces or engagement by means of compression using a rubber ring or other means may be employed. The connector 123 may have, on the side surface thereof, a rib or a dent fitting a finger so as to be easily gripped when attached to the opening 213.

While a configuration has been described in which the inner surface of the connector 123 and the outer surface of the opening 213 have the guide mechanism, the outer surface of the connector 123 and the inner surface of the opening 213 may have the guide mechanism. For example, the outer surface of the connector 123 and the inner surface of the opening 213 may have a guide mechanism, such as screw threads.

It is preferred that the connector 123 and the opening 213 are connected not in an airtight manner so that the inside of the shell 201 is not airtight when the connector 123 is attached to the opening 213. A ventilation path may pass between the connector 123 and the opening 213. For example, the connector 123 or the opening 213 may have a ventilation groove extending in the axial direction so as to intersect the corresponding screw thread, and a ventilation path may appear when the connector and the opening are threadedly engaged. The cap 102 may have a vent hole, for example, not to make the inside of the shell 201 airtight. The shell 201 may have a vent hole.

If the connector 123 has a screw within a specification (i.e., a universal joint) of the opening of a typical bottle for beverage, food, or other substances, the shell 201 can be manufactured in the same process as a widely distributed beverage containers. The shell 201 within the same specification as the beverage containers is easily recycled and is thus advantageous in reducing waste. In an emergency, a beverage bottle or other containers (e.g., a PET bottle made of polyethylene terephthalate) can be used as the shell 201, which improves the convenience.

The connector 123 may be connected only to the opening 213 of a dedicated shell 201. In this case, the connector 123 connectable only to the dedicated shell 201 designed in accordance with the size of the balloon body 101 can reduce the following circumstances. A too-small shell 201 fails to inflate the balloon body 101 sufficiently. A too-large shell 201 fails to stabilize the balloon body 101 inside.

The shell 201 as a dedicated product may have an indicator, such as a scale, serving as a guide of the degree of inflation of the balloon body 101. This allows the user to visually grasp an approximate amount of the drug solution in the balloon body 101. The opening of the shell 201 may have an inner diameter that allows a non-inflated balloon body 101 and disallows an inflated balloon body 101 to pass. This makes it difficult to pull out the balloon body 101 from the hard shell 201 in use, which can reduce the erroneous use by the user.

In this embodiment, the balloon body 101 is in a tubular shape with one end sealed and fixed to the cap 102 by a fastening member 127, and the other end sealed. The inlet port 121 communicates with the inner cavity of the balloon body 101 via an inlet flow passage having a check valve, and the outlet port 122 communicates with the inner cavity of the balloon body 101 via an outlet flow passage. The drug solution introduced through the inlet port 121 is reserved in the balloon body 101, but led out not through the inlet port 121 due to the presence of a check valve but through the outlet port 122.

A pipe shaped shaft 125 protruding beyond the cap is disposed in the balloon body 101. With the shaft 125 provided, the balloon body 101 can inflate uniformly and reduce the amount of the drug solution remaining inside when deflating. In addition, with the shaft 125 provided, an insertion operation of inserting the balloon body 101 into the shell 201 becomes easier.

The balloon body 101 may be made of an elastic body, such as rubber or elastomer. While being sealed by welding the distal end away from the cap 102 in this embodiment, the balloon body 101 may be molded in the shape of a closed tube. Alternatively, a member, such as a plug, for closing the distal end may be used for the sealing. The proximal end of the balloon body 101 is fixed to the fastening member 127 of the drug solution passage 120. In this embodiment, the fastening member 127 includes an inner cylinder 127A connected to the inlet port 121, and an outer cylinder 127B surrounding the inner cylinder 127A. The outer cylinder 127B presses the balloon body 101 externally fitted to the inner cylinder 127A from the outside. Not limited to such the method, the balloon body 101 can be fixed to the drug solution passage 120 by various methods.

The shell 201 may be made of any of various resin materials. For example, when the shell 201 is a hard shell, the shell 201 may be made preferably of a resin material, such as polyethylene terephthalate (PET), polyethylene (PE), polypropylene (PP), acrylic, or acrylonitrile-styrene (AS), harder than the balloon body 101. When the shell 201 is a soft shell, the opening and the cylinder for housing the balloon body 101 may be made of different materials. In this case, the opening is preferably made of a hard material and the cylinder is preferably made of a soft material. The entire body can be however made of a soft material with an adjustable thickness, for example. As the soft material, soft vinyl chloride, polyurethane, or any other suitable material may be used. The shell 201 may be transparent to allow the user to check the state of the balloon body 101 therein. Depending on the type of the drug solution to be reserved, the shell 201 may have light shielding properties not to transmit light or disallow the light with a specific wavelength to pass.

While being a female lure connector to which a syringe barrel with a lure lock is connectable in this embodiment, the inlet port 121 may have various configurations to which various instruments for injecting a drug solution is connectable. While the outlet port 122 is a tube port to which a tube can be welded and fixed, the configuration is not limited thereto and may be any of various configurations. While an example where the balloon 100 has the inlet port 121 and the outlet port 122 has been described, the balloon 100 may have one port. In this case, for example, a three-way connector having a check valve or a stopcock may be connected to the port to introduce and discharge the drug solution. As a possible configuration, the operation of introducing the drug solution from the inlet port 121 may be difficult unless the connector 123 is connected to the shell 201. With this configuration, the use without the balloon body 101 inserted into the shell 201 can be reduced.

The shell 201 may be integrally molded, but, for example, the opening 213 molded separately may be fixed to the shell body 211. In this manner, when the size of the shell 201 is changed in accordance with the capacity of the balloon body 101, the opening 213 can be shared easily. In addition, the strength of the opening 213 can be increased easily. The opening 213 may be integrated with the shell body 211 by two color molding, for example.

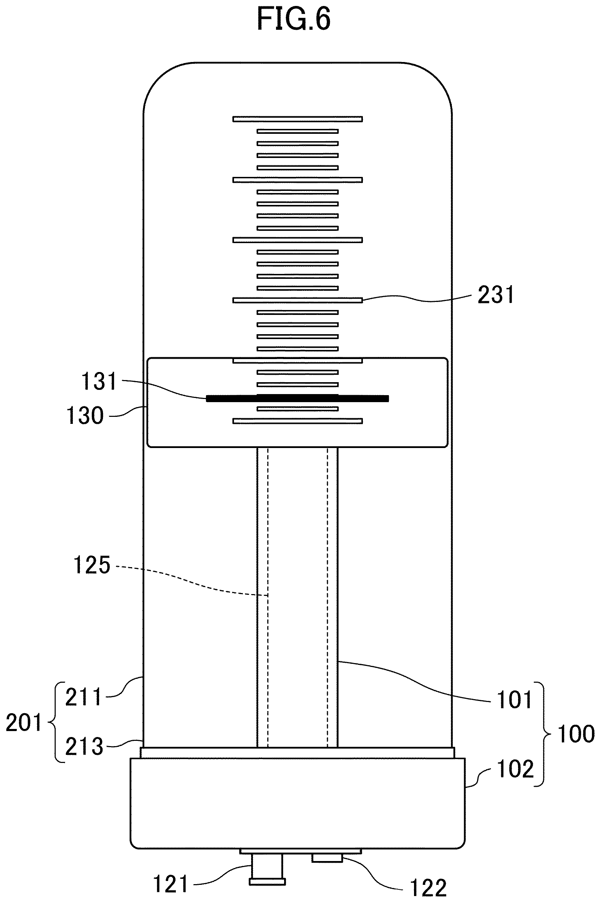

With the inner diameter of the opening 213 increased to some extent, the balloon body 101 is inserted more easily. Accordingly, the inner diameter of the opening 213 is preferably 1/2 or more the inner diameter of the shell body 211. On the other hand, reducing the diameter of the opening 213 of the shell 201 to some extent and reducing the diameter of the connector 123 of the cap 102 make it possible to make the balloon 100 compact enough, thereby reducing the volume of the medical waste. However, as shown in FIG. 6, the opening 213 and the shell body 211 may have substantially the same diameter so that the shell 201 becomes in a cylindrical shape. The shell 201 in a cylindrical shape has a large opening 213, and the balloon body 101 is thus inserted easily. In addition, the molding die becomes simple, which facilitates the production.

In the case of a cylindrical shell 201, the balloon body 101 provided with a movable portion 130 having an outer diameter slightly smaller than the inner diameter of the shell body 211 can be easily inserted into the shell 201. The movable portion 130 with an outer diameter substantially equal to the inner diameter of the shell 201 moves together with the distal end of the balloon body along the inner surface of the shell 201 when the balloon body 101 inflates and deflates. Accordingly, by providing a gridline 131 on the movable portion 130 and a scale 231 on the shell 201 and reading the point where the gridline 131 of the movable portion 130 and the scale 231 of the shell 201 overlap, the user knows the approximate amount of the drug solution reserved in the balloon body 101. The movable portion 130 may be made of a deformable soft material to be insertable from the opening 213 having a small diameter.

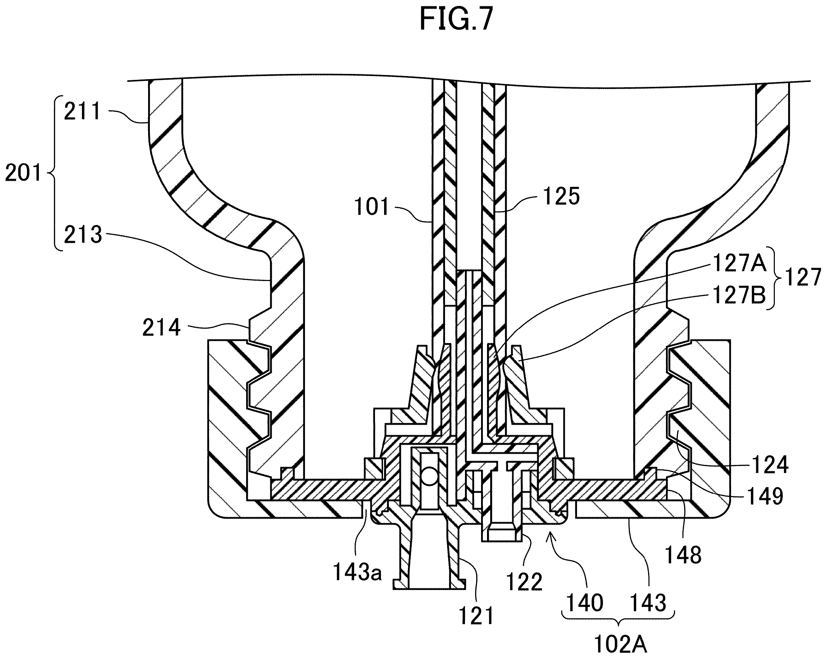

A cap 102A may be attached to the opening 213 by a connector 143 independent from the other parts. For example, as shown in FIG. 7, the cap 102A may include a drug solution passage 140 having a flange 148 and the connector 143 having an opening 143a at a central portion. By aligning the drug solution passage 140 with the opening 213 of the shell 201 and threadedly engaging the connector 143 to the opening 213, the flange 148 can be sandwiched and fixed between the opening 213 and the connector 143. Specifically, the flange 148 has a sandwiched portion extending in the radial direction and sandwiched between the connector 143 and the shell 201 in the vertical direction.

Being separated from the drug solution passage 140, the connector 143 becomes reusable, which can further reduce the amount of regulated medical waste. The sandwiched portion among the shell 201, the drug solution passage 140, and the connector 143 is not limited to the flange 148, and may be any of various members sandwiched between the opening 213 and the connector 143, for example. How to connect the drug solution passage 140 to the shell 201 is not limited to sandwiching the portion between the opening 213 and the connector 143 and any of various methods may be employed.

In FIG. 7, the flange 148 has a projection 149 as an aligner which is engaged with a recess of the opening 213 to assist the alignment of the shell 201 and the drug solution passage 140, or to temporarily fix the shell and the drug solution passage 140 not to cause misalignment after the alignment. This can greatly improve the handleability. The projection and the recess as the aligners may be in an annular shape, may include a plurality of projections and a plurality of recesses independently, or may be only one of a projection or recess. The flange 148 may have a recess, and the opening 213 may have a projection. The aligners are not limited to the projection and recess and may have any of various configurations. In addition, not only the flange 148, but also any of various configurations capable of aligning a certain part of the drug solution passage 140 with the opening 213 may be employed. Note that the aligners may be provided as necessary and are not necessarily provided.

The connection between the connector 143 and the opening 213 is not limited to threadedly engaging and can be achieved by any of various configurations capable of detachably engaging these as described above. In addition, the connector 143 may be attached to the opening 213 after engaging the drug solution passage 140 and the connector 143 with each other. For example, the outer diameter of the flange 148 may be substantially equal to the inner diameter of the connector 143 so that the flange 148 can be engaged with the connector 143. Then, the connector can be attached to the opening 213 after being integrated with the drug solution passage 140.

Even if the drug solution passage 140 and the connector 143 are independent from each other, various configurations may be provided as a ventilation path communicating with the outside so that the inside of the shell 201 to which the balloon 100 is attached is not airtight, as in the case where the drug solution passage 140 and the connector 143 are integral with each other. Example configurations include a configuration where a ventilation path is formed between the connector 143 and the flange 148, a configuration where a ventilation groove is formed between the opening 213 and the flange 148, and a configuration where a through-hole is provided in the flange 148.

If the inner diameter of the opening 213 is smaller than the shell body 211, the balloon body 101 may inflate insufficiently at the opening 213. In such a case, a largely inflating portion of the balloon body 101 can be positioned inside the shell body 211 beyond the opening 213. For example, if the distal end of the balloon body 101 inflates more than the fastening member 127, the distal end of the fastening member 127 may be positioned closer to the shell body 211 beyond the opening 213.

By changing the size of the balloon body 101, various balloons 100 for a balloon infuser having different drug solution reservoir capacities can be achieved. In addition, it is possible to achieve balloon infusers with various characteristics, such as flow rates, as well as reservoir capacities. In this case, in order to easily distinguish the balloons 100 with different reservoir capacities or other characteristics, an indicator 138 for indicating at least one of the capacity, the flow rate, the rod number, the item number, or any suitable number, by means of a color, a character, a symbol, or any other information.

The indicator 138 is not particularly limited. For example, if located on the outer surface of the connector 123, the indicator 138 can be less likely to be hidden even after being attached to the shell 201. While an example where the indicator 138 is located on the side surface of the connector 123 has been shown in FIG. 1, the indicator 138 may be located on the top of the connector 123. The indication may be made not only on the connector 123 but also on another portion. For example, the indication may be made on the balloon body 101 or by changing the color of the entire balloon body 101. In the case of the reusable connector 143, the indication may be made on a non-reusable portion, such as the drug solution passage 140 of the flange 148, or on the balloon body 101. The indicator may also be achieved by changing the color of any port and/or flange. At least some of the indications indicating the capacity, flow rate, lot number, and item number may be located on the balloon 100 at the same point together or separately at different points. These indications can be each made by attaching a label, printing on the surface of any member, or coloring any member.

If the balloon 100 including the balloon body 101 with a large capacity is combined with a small shell 201, the balloon body 101 cannot inflate to a specified size in the shell 201, which causes an improper amount of the drug solution to be reserved. In addition, the shell and the balloon may interfere with each other, which may cause burst of the balloon.

In order to reduce such circumstances, the shell 201 may also have an identifier 238 by means of a color or a mark. In the embodiment shown in FIG. 1, the indicator functions as both the capacity indicator and the identification indicator, and also functions as an identification correspondence portion. By matching the indicator 138, which is the identification correspondence portion, on the balloon 100 and the identifier 238 on the shell 201, whether the combination is proper can be checked easily and visually. It is possible to easily and visually check whether the combination is proper by making the identifier on the shell 201 identical to the identification correspondence portion on the balloon 100. However, the identifier and the identification correspondence portion are not necessarily identical, as long as whether the combination is proper can be checked. While FIG. 1 shows an example where the identifier 238 is located on the outer surface of the shell 201, the location of the identifier on the shell 201 is not particularly limited. For example, when the connector 123 has the capacity indicator of the balloon 100, the identifier of the shell 201 may be located on the opening 213. By making the indication on the mutually engaging portions, whether the combination is proper can be determined easily.

The identifier and the identification correspondence portion may be of various types that are visually checkable and can match each other by means of the same color, character, and/or symbol, for example. A plurality of these may be used. The symbol may be a circle, a triangle, or a cross, for example.

The identifier and the identification correspondence portion may be aligned in a line, for example, when the balloon infuser is attached to a proper shell, or may be in the same shape and/or color. The identifier and the identification correspondence portion are not necessarily in the same. For example, one of them may be a line and the other may be aligned with the line in the case of a proper or improper combination. The indication may be made by attaching a label, printing on the surface of any member, or coloring any member. If the connector 143 and the drug solution passage 140 are separate bodies, the drug solution passage 140 may have the identification correspondence portion. For example, the identification correspondence portion may be a flange in the same color as the identifier. Alternatively, the gridline 131 on the movable portion 130 and the scale 231 on the shell may match each other in the same color, as the identifier and the identification correspondence portion. For example, the letters "OK" may appear on the window when the balloon infuser is attached to a proper shell, and "NG" on the window when the balloon infuser is attached to an improper shell.

Instead of using the same color, for example, the size of the opening of the shell may be treated as the identifier, and the size of the connector as the identification correspondence portion. Not only the sizes of the connector 123 and the opening 213, various configurations capable of attaching the balloon 100 only to the associated shell 201 may be employed. Even a configuration where physical connection is not possible, a capacity indication may be made by means of a color, for example.

While an example has been described where the outlet port 122 is a tube fixable by adhesion or other means, a tube may be detachably connected via a connector. With this configuration, the user can connect the tube to the outlet port after inserting the balloon body 101 into the shell 201, and thus the tube does not interfere when the balloon body 101 is inserted into the shell 201. **In** addition, the user can select and connect a tube with a most proper length. If the tube is fixed to the outlet port 122, a tube holding clip for temporarily fixing the tube to the connector 123 can be provided so that the tube does not interfere when the balloon body 101 is inserted into the shell 201.

The outlet port 122 may have a valve body, such as a duckbill valve, opened by connecting a connector. This configuration can eliminate the need to close the tube at the outlet using a clamp or any other suitable means when the ballon body is filled with the drug solution, which improves the handleability.

The balloon 100 for a balloon infuser can be contained in a normal sterilization bag or any other suitable item having a gas permeable portion and subjected to ethylene oxide gas sterilization or high-pressure vapor sterilization. The sterilization bag has a structure, such as a valve, or a gas flow path, includes a sterilization paper, which is nonwoven paper disallowing bacteria to pass, or partially serves sterilization paper. Without putting in a bag, the inlet port 121 and the outlet port 122 may be covered with an air-permeable cover member and subjected to sterilization. The cover member may be a gas-permeable adhesive member that can be attached to the port. The adhesive member may be a porous film or a sterilization paper, for example, disallowing bacteria or other contaminants to pass. Without using any bag, waste can be reduced. The port may be sealed and sterilized, and then contained in a bag, a tray, or any other suitable container.

If the port is sealed with an adhesive member or any other suitable means and sterilized, the user can easily confirm that the product is unused with the inside sterilized, by means of the sealing. By unsealing, the top of the port appears, which makes it visible at a glance that the product has been used. A part of the seal may remain or a trace of the seal may appear after unsealing. This can encourage the user no to reuse the used product.

A used-state indicator indicating that the product has been used can be provided separately. For example, the used product may be identifiable by providing a cover on the connector 123 or the balloon body 101 and removing the cover for use. A used product may be identifiable by changing the color of a part of the balloon infuser or partially breaking the balloon infuser when removing the cover.

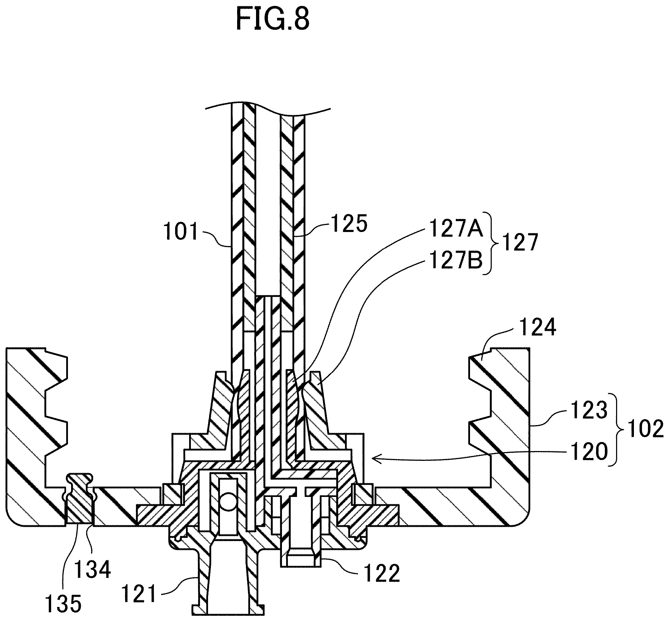

The indicator may appear when the connector 123 is connected to or disconnected from the opening 213. For example, as shown in FIG. 8, the connector 123 may have, on the top thereof, an opening 134 into which a pin member 135 is inserted. Once the connector 123 is connected to the opening 213, the pin member 135 is pushed into the opening 134 by the opening 213 and appears beyond the top of the connector 123. Since the end of the inner surface of the opening 134 is a narrow portion over which the proximal end of the pin member 135 can ride, the pin member 135 pushed into the opening 134 is locked. Even when the connector 123 is disconnected from the opening 213, the pin member 135 is kept protruding and thus functions as a used-state indicator. This allows the user to easily check whether the product is used. The location, size, and shape of the pin member may be modified variously. The portion of the pin member 135 exposed inside the shell 201 is in a rod shape in FIG. 8, but may be in a ring shape to be mountable on the top of the opening 213 of the shell 201.

The pin member 135 may be colored differently from the top of the connector 123 so that the appearance of the pin member 135 can be recognized more easily. When the pin member 135 appears by adjusting its location or protrusion length, the pin member 135 may interfere with the connector to be connected to the inlet port 121 so that the connector cannot be connected to the inlet port 121. It is also possible to configure the pin member 135 to protrude above the inlet port 121 so as to prevent a syringe from being attached to the port. In these cases, the balloon 100 is inserted into the shell 201 for use after the balloon body 101 is filled with the drug solution.

The detent mechanism not to allow the appearing used-state indication to return may be obtained by, for example, providing a triangular or semicircular projection, over which the pin member can ride, on the path of the pin member that moves when the connector 123 becomes closer to the opening 213 and connected. The detent mechanism disallows the pin member beyond the projection to return to the state before use.

The indicator indicating that the product has been used can be formed by providing portions that come into contact with each other when the connector 123 and the opening 213 are connected to each other, and disposing a pressure-sensitive film, for example, on which another color appears under the pressure applied to the contact. The indicator may have a mechanism in which an indicator indicating that the product has been used appears when the connector 123 is connected to or disconnected from the opening 213, or may be configured such that a part of the connector 123 is bent or damaged to indicate that the product has been used. The indicator may also be provided with a mechanism in which reconnection is physically prevented by bending or damaging the connector 123 once connection is made. The indicator may also be configured such that a part of the connector 123 is broken and remains on the opening 213 when the connector 123 is disconnected from the opening 213. The part to be broken may be in a semicircular shape or a C-shape to be easily removable from the opening 213.

While an example has been described where the bottom of the shell 201 away from the opening 213 is flat, the bottom is not necessarily flat and may curve. With the flat bottom, the shell 201 may stand upright. While an example has been described where the bottom of the shell 201 is closed, the bottom may be open. In this case, the balloon 100 may be inserted not through the opening 213 but through the open bottom. If the balloon 100 includes the movable portion 130, the insertion can be made easily through the open bottom. As necessary, a lid or any other suitable means for closing the opening of the bottom of the shell 201 may be attached in use.

### INDUSTRIAL APPLICABILITY

The balloon for a balloon infuser according to the present disclosure can solve the problems of the storage space and an increased waste, while ensuring the safety and reducing the risk of erroneous use, and is useful in the medical field.

### DESCRIPTION OF REFERENCE CHARACTERS

- 100: Balloon for Balloon Infuser
- 101: Balloon Body
- 102: Cap
- 102A: Cap
- 120: Drug Solution Passage
- 121: Inlet Port
- 122: Outlet Port
- 123: Connector
- 124: Female Screw
- 125: Shaft
- 127: Fastening Member
- 127A: Inner Cylinder
- 127B: Outer Cylinder
- 128: Projection
- 130: Movable Portion
- 131: Gridline
- 134: Opening
- 135: Pin Member
- 138: Indicator
- 140: Drug Solution Passage
- 143: Connector
- 143a: Opening
- 148: Flange
- 149: Projection
- 151: Projection
- 152: Flexible Piece
- 201: Shell
- 211: Shell Body
- 213: Opening
- 214: Male Screw
- 215: Groove
- 216: Protrusion
- 221: Projection
- 231: Scale
- 238: Identifier

## Claims

1. A balloon for a balloon infuser, the balloon comprising:
a balloon body that is insertable into a shell and that reserves and pumps out a drug solution;
a drug solution passage fixed to one end of the balloon body and provided with a flow path through which the drug solution is introduced into and discharged from the balloon body; and
a connector detachably connecting the balloon body to the shell while the ballon body is located in the shell, the balloon body being removable from the shell by disconnecting the connector from the shell.

2. The balloon of claim 1, wherein
the connector has a guide structure that guides movement relative to the shell in an axial direction.

3. The balloon of claim 2, wherein
the guide structure is a connector-side screw to be threadedly engaged with an opening-side screw at the opening of the shell.

4. The balloon of any one of claims 1 to 3, wherein
the connector has a lock mechanism that restricts disconnection from an opening of the shell.

5. The balloon of any one of claims 1 to 4, wherein
the connector includes a flexible piece and a projection at a free end of the flexible piece, and
the connector is connected to the shell by bringing the connector close to the shell so that the projection rides over a shell-side projection of the shell.

6. The balloon of any one of claims 1 to 5, wherein
the connector is integrally molded with the drug solution passage.

7. The balloon of any one of claims 1 to 5, wherein
the connector is separable from the drug solution passage.

8. A balloon for a balloon infuser used in combination with a shell and a connector, the balloon comprising:
a balloon body that is insertable into the shell and reserves and pumps out a drug solution; and
a drug solution passage fixed to one end of the balloon body and provided with a flow path through which the drug solution is introduced into and discharged from the balloon body, wherein
the drug solution passage includes a portion sandwiched between the shell and the connector,
the balloon body is held inside the shell by connecting the connector to the shell, and the balloon body is removable from the shell by disconnecting the connector from the shell.

9. The balloon of any one of claims 1 to 8, further comprising:
an indicator that indicates characters indicating at least one of a lot number, an item number, a capacity of the balloon body, or a flow rate.

10. The balloon of any one of claims 1 to 9, further comprising:
an identification correspondence portion corresponding to an identifier in the shell, wherein
the identifier and the identification correspondence portion indicate that at least a capacity of the balloon body matches a volume of the shell.

11. The balloon of any one of claims 1 to 10, further comprising:
a used-state indicator that appears by connecting or disconnecting the connector to or from the shell and indicates that the balloon has been used.

12. The balloon of claim 11, wherein
the used-state indicator has a detent mechanism that restricts return of the used-state indicator.

13. A balloon infuser comprising:
a shell; and
a balloon for the balloon infuser, the balloon including a balloon body that reserves and pumps out a drug solution, a drug solution passage fixed to one end of the balloon body and provided with a flow path through which the drug solution is introduced into and discharged from the balloon body, and a connector detachably connecting the ballon body to the shell while the ballon body is located in the shell,
wherein the balloon body is removable from the shell by disconnecting the connector from the shell.
